# EUROPEAN PATENT APPLICATION

(11) **EP 3 385 697 A1**
(43) Date of publication of application: **10.10.2018**
(21) Application number: 17382176.0
(22) Date of filing: 04.04.2017
(51) Int. Cl.: G01N 1/28, G01N 21/65, B01L 3/00, G01N 1/31, G01N 21/03, G01N 33/50

(54) **SAMPLE HOLDER**

(71) Applicant: Raman Health Technologies, S.L, 47151 Boecillo (ES)
(72) Inventor: CATALÁ ESPÍ, Alejandro, E-47151 Boecillo, Valladolid (ES); VENEGAS DEL VALLE, Gloria, E-47151 Boecillo, Valladolid (ES); ZANOTTI, Carlo, E-47151 Boecillo, Valladolid (ES)
(74) Representative: ABG Patentes, S.L.

(57) **Abstract**

A sample holder (100) comprising a main body (1) comprising a first cavity (7), the first cavity comprising a bottom and an open end, a sample supporting element (2) comprising a receiving surface made of metal, the sample supporting element having a first through hole (8) arranged at the receiving surface, and a paper filter (3), wherein the sample supporting element and the paper filter are stacked inside the first cavity of the main body, the sample supporting element being arranged on the paper filter and such that the receiving surface of the sample supporting element is accessible through the open end of the first cavity.

## Description

### TECHNICAL FIELD OF THE INVENTION

The present invention belongs to the field of devices used for the analysis of liquid samples, preferably liquid biological samples. More particularly, the present invention belongs to the field of sample holders used in in vitro analysis, for example in Raman spectroscopy.

### BACKGROUND OF THE INVENTION

In the field of analysis of liquid samples, a sample holder is required for the support of the sample in optimal conditions. Other important aspect is the spatial distribution of the liquid sample on the sample holder, because it may influence the reliability of the analysis of the sample.

In the technical field of micro Raman analysis, liquid biological samples need to be extended and dried on the surface of a supporting element. In the case of plasma, rapid drying is critical in order to avoid changes in plasma components, such as proteins and small molecules.

A known method to perform sample extension of a liquid sample is by manually extending the sample with a spreader, such as the same element used for the deposition of the sample drop on the supporting element, a slide or a swab. The accuracy of this method relies on the expertise of the person performing the sample extension. Thus, the problem with this known method is that it is very user dependent and lacks reproducibility and inter-sample repeatability.

Other methods known in the state of the art use automatic smear devices including moving parts. However, these devices are complex and costly.

In the field of Raman analysis of biological samples, it is required that the material of the sample supporting element has a Raman response not overlapping the spectrum of the biological sample. A limited range of materials can be used depending on the region of interest of the Raman bands backscattered by the sample.

Document US 8835128 B2 discloses an identification method based on surface-enhanced Raman scattering (SERS), in which bacterial samples are used in identification of bacteria by mixing with concentrated silver and gold nanoparticles. This document describes a method for manually extending a sample using moving parts. Thus, the method requires the manual use of moving parts and therefore it also depends on the skills of the person performing the sample extension.

Document EP 1271125 B1 discloses an automatic smear preparing apparatus including a sample smearing section for smearing a biological sample on a slide glass by using a spreader glass. However, this apparatus requires a complex mechanical system. Also, some of the moving parts of the device are in contact with the supporting element and thus contamination of the sample and of the moving parts themselves cannot be avoided.

It is therefore an object of the invention to provide a sample holder which solves the above cited problems.

### SUMMARY OF THE INVENTION

The present invention provides a solution to the aforementioned problems, by a sample holder according to claim 1, a kit according to claim 13 and a method for depositing and extending a sample according to claim 15. In dependent claims, preferred embodiments of the invention are defined.

In a first inventive aspect, the invention provides a sample holder comprising:
a main body comprising a first cavity, the first cavity comprising a bottom and an open end,
a sample supporting element comprising a receiving surface made of metal, the sample supporting element having a first through hole arranged at the receiving surface, and
a paper filter.

The sample supporting element and the paper filter are stacked inside the first cavity of the main body, the sample supporting element being arranged on the paper filter and such that the receiving surface of the sample supporting element is accessible through the open end of the first cavity. In other words, the sample supporting element is arranged with its receiving surface facing outwards the first cavity and with the surface opposite the receiving surface contacting the paper filter.

The first cavity of the main body is configured to house a plurality of elements. Said elements are stacked forming a stacked structure. The stacked structure is inserted in the first cavity, preferably pressure-inserted. Therefore, one end of the stacked structure is arranged on the bottom of the first cavity and the opposite end of the stacked structure is arranged at the open end of the first cavity.

In this embodiment the sample supporting element and the paper filter are the elements forming the stacked structure. The paper filter rests at the bottom of the first cavity and the sample supporting element is arranged on the paper filter in the first cavity, such that the receiving surface of the sample supporting element is accessible through the open end of the first cavity. In other embodiments additional elements may be included in the stacked structure.

Preferably, the sample supporting element and the paper filter have a substantially flat configuration.

The sample supporting element has a first through hole configured to allow percolation by capillarity of a sample drop deposited on the receiving surface, such that the sample is transferred from the receiving surface to the opposite side of the sample supporting element through the first through hole. Preferably, the first through hole is arranged at a position of the receiving surface substantially corresponding to a centre of symmetry of the sample supporting element.

Along the description, the term liquid sample should be understood as a sample that is substantially in liquid state. This also includes any liquid comprising any level of viscosity which can be transferred through capillarity from one surface to another surface.

The paper filter is positioned just below the sample supporting element and is configured to break the surface tension of the sample drop and to absorb the excess of liquid sample deposited on the receiving surface and coming through the first through hole.

In a use situation of the sample holder according to the invention, when a drop of a liquid sample is deposited on a region of the receiving surface where the first through hole is located, the liquid goes through the first through hole by the action of gravity and capillarity and passes from the receiving surface to the opposite side of the sample supporting element. Once the liquid arrives at the opposite side of the sample supporting element, the liquid touches the paper filter, which absorbs the liquid sample until the first through hole of the sample supporting element is emptied. As a result, a thin layer of liquid sample is kept extended on the receiving surface and the remainder liquid is absorbed by the paper filter arranged below the sample supporting element. This advantageously allows a fast drying of the liquid sample located on the receiving surface.

Advantageously, with the sample holder of the present invention the sample drop is distributed across the surface of the sample supporting element opposite the receiving surface, with the action of only gravity and capillarity. Thus, the sample holder of the invention allows automatic, uniform and reproducible extension of liquid samples without the use of mobile elements or mechanical apparatus, either simple or complex, and without user intervention. Finally, the sample holder advantageously allows its use for Raman spectroscopy, since metals or metallic surfaces are not active in Raman and thus the sample holder of the invention does not interfere with the Raman spectrum of the sample.

In an embodiment of the invention, the sample supporting element is arranged in the first cavity such that the receiving surface protrudes from the surface of the main body. Advantageously, this embodiment guarantees that the objective of a microscope used in a subsequent analysis of the sample does not encounter any obstacle, thus allowing access to the whole extended sample.

In an embodiment of the invention, the sample holder further comprises a cover attachable to the main body. The cover comprises a second through hole and is configured such that when the cover is attached to the main body, the second through hole and the first through hole are aligned.

The cover is configured to guide any instrument configured to deliver a sample, such as a pipette, to a specific delivery point defined by the second through hole, which is located above the receiving surface of the sample supporting element and aligned with the first through hole. It is thus guaranteed that a sample delivered through the second through hole will reach the receiving surface of the sample supporting element at the position of the first through hole. Advantageously, the cover also reduces the risk of contamination by a user touching the main body.

In an embodiment of the invention, the cover is configured such that when the cover is attached to the main body the second through hole is kept spaced from the first through hole. According to this embodiment a sample delivered through the second through hole will be delivered at a distance from the receiving surface as defined by the cover, thus improving sample extension reproducibility and inter-sample repeatability, since the distance between pipette and receiving surface has an effect on the size of the sample drop reaching the receiving surface. This is important for applications where repeatability of sample extension is crucial for the accuracy of subsequent analysis. In this embodiment the cover provides a spacing between the cover and the receiving surface. The spacing between the cover and the receiving surface allows the end of a pipette to deliver a drop increasing in size as the pipette is pressed, the drop reaching a sufficiently large diameter before the drop is separated from the pipette due to the action of gravity. Advantageously, an increased drop diameter projected onto the receiving surface is achieved and the sample is extended over an increased area.

In an embodiment of the invention where the cover is configured such that when the cover is attached to the main body the second through hole is kept spaced from the first through hole, the cover further comprises a tunnel configured to allow flow of air along the receiving surface of the sample supporting element. Advantageously, the tunnel is configured to let a laminar flux of air flow along the receiving surface of the supporting element, thus enhancing the drying speed of the sample.

In an embodiment, the spacing between the second through hole and the first through hole when the cover is attached to the main body is in the range from 7 mm to 15 mm. Advantageously, this range assures that a diameter of the drop deposited onto the receiving surface is large enough to leave sufficient area of sample around the first through hole, while allowing avoiding a drop of the sample sticking to the base of the cover.

In an embodiment of the invention, the cover further comprises a cap configured to be removably plugged to the second through hole. Advantageously, the cap avoids particles falling onto the deposited sample, preventing the contamination of the sample by any external agent.

In an embodiment of the invention, the cover and/or the main body further comprise at least one fixing element configured to attach the cover to the main body. In a particular embodiment, the fixing element is at least one protrusion arranged on the cover and at least one recess arranged on the main body and such that the protrusions of the cover are receivable in a corresponding recess of the main body. In other embodiment, the protrusions may be arranged at the main body and the corresponding recesses may be arranged on the cover.

In an embodiment of the invention, the sample holder comprises a test paper configured to change its colour when the test paper is in contact with a sample. In this embodiment the test paper is stacked inside the first cavity, the paper filter being placed between the test paper and the sample supporting element, such that the test paper is arranged facing the bottom of the first cavity.

In this embodiment, the stacked structure comprises three elements: the sample supporting element, the paper filter and the test paper. Said elements are stacked and inserted into the first cavity, preferably pressure-inserted. In this embodiment, the test filter rests on the bottom of the first cavity and the sample supporting element is arranged at the open end of the first cavity, with the paper filter being placed between the test paper and the sample supporting element. The sample supporting element is arranged such that the receiving surface is accessible through the open end of the first cavity.

In this embodiment, the test paper is placed below the paper filter and has a double purpose: to further absorb the excess of liquid sample and to change colour after getting in contact with this excess of sample, which reflects the pH of the sample. Additionally, colouring of the test paper serves to indicate that the extension of the sample has been successfully accomplished.

Preferably, the sample supporting element, the paper filter and the test paper have a substantially flat configuration.

In an embodiment of the invention, the test paper is substantially round-shaped and its diameter is in the range from 5 mm to 15 mm.

In an embodiment, the thickness of the test paper is in the range from 10 micrometres to 30 micrometres.

In an embodiment of the invention, at least a part of the main body corresponding to the first cavity is made of transparent material, such that the paper filter and/or the test paper housed in the interior of the first cavity are visible through the main body. Advantageously, this embodiment allows the technician to monitor the deposition of the sample from the outside, by simply observing through the transparent main body the absorption of the sample by the paper filter and/or the test paper, without contaminating said sample. In an embodiment the whole main body is made of transparent material.

In an embodiment of the invention, the receiving surface is made of a biocompatible metal. Advantageously, this embodiment allows the sample holder of the invention to be used to hold liquid biological samples. In a particular embodiment, the biocompatible metal is gold, aluminium, brass, copper, titanium or stainless steel.

In an embodiment the whole sample supporting element is made of metal, preferably a biocompatible metal. In other embodiment only the receiving surface is made of metal, such as a metal coated or plated on the material of the sample supporting element. Preferably, the metal is a biocompatible metal.

In a preferred embodiment, the biocompatible metal is gold. Advantageously, gold has the following advantages:
- Gold does not oxidize.
- Gold has excellent thermal conductivity, which allows for faster drying of the liquid sample.
- Gold intensifies Raman signal during the analysis of the extended sample.

In a preferred embodiment the sample supporting element is made of brass and the receiving surface is gold-plated. Preferably, the thickness of the gold-plated layer is in the range from 0.5µm to 5µm.

In an embodiment the receiving surface is polished or jewellery-polished, which enhances Raman response.

In an embodiment of the invention, the sample supporting element is substantially round-shaped and has a diameter in the range from 10 mm to 20 mm. The first through hole is preferably arranged substantially at the centre of the sample supporting element.

In an embodiment of the invention, the paper filter is substantially round-shaped, and has a diameter in the range from 14 mm to 30 mm.

In an embodiment the thickness of the paper filter is in the range from 10 micrometres to 60 micrometres.

In an embodiment the thickness of the sample supporting element is in the range from 1 mm to 5 mm.

In an embodiment the diameter of the first through hole is in the range from 1 mm to 5 mm.

In a second inventive aspect, the invention provides a kit comprising a sample holder according to any of the embodiments according to the first inventive aspect and a pipette.

Advantageously the kit of the invention provides an extension of a liquid sample in the sample holder according to the invention without any mobile elements or mechanical apparatus, enhancing the reproducibility and avoiding the user intervention for obtaining an extended dried sample.

In an embodiment of the kit, the sample holder is according to any embodiment of the first inventive aspect where the sample holder comprises the cover, and the cover of the sample holder and the pipette are configured such that when the pipette is inserted in the second through hole of the cover the distance between the delivery end of the pipette and the sample receiving surface is in the range from 5mm to 10mm. The delivery end of the pipette will be understood as the end of the pipette from which a sample drop leaves the pipette.

In a particular embodiment, the volume of the pipette is in the range of 20 microliters to 100 microliters.

In a third inventive aspect, the invention provides a method for depositing and extending a sample comprising the following steps:
a) providing a kit according to any of the embodiments of the second inventive aspect, when the sample holder is according to any embodiment of the first inventive aspect when said sample holder comprises the cover,
b) loading the sample in the pipette,
c) inserting the pipette in the second through hole of the cover,
d) pressing the pipette to deliver a drop of sample,
e) once the drop of sample has been deposited onto the receiving surface of the sample supporting element, extracting the pipette from the cover,
f) waiting until the first through hole is emptied and the sample remaining on the receiving surface is dried.

Advantageously, the method is cost-effective, simple, repeatable and operator independent and allows a fast drying liquid sample deposition and self-extension on the receiving surface of the sample holder according to the invention.

All the features described in this specification (including the claims, description and drawings) and/or all the steps of the described method can be combined in any combination, with the exception of combinations of such mutually exclusive features and/or steps.

### DESCRIPTION OF THE DRAWINGS

These and other characteristics and advantages of the invention will become clearly understood in view of the detailed description of the invention which becomes apparent from a preferred embodiment of the invention, given just as an example and not being limited thereto, with reference to the drawings.
Figure 1 shows an exploded view of an embodiment of a sample holder according to the invention.
Figure 2a shows a front view of the sample holder of figure 1.
Figure 2b shows a front view of the sample holder of figure 1 wherein its internal structure is denoted with dashed lines.
Figure 3 shows a top view of the sample holder of figure 1.
Figure 4 shows an embodiment of a pipette of a kit according to the invention.
Figure 5a shows an embodiment of a kit according to the invention.
Figure 5b shows the kit of figure 5a wherein its internal structure is denoted with dashed lines.
Figure 6a shows a sectional view of the kit of figures 5a and 5b.
Figure 6b shows the sectional view of figure 6a further showing the internal structure, which is denoted with dashed lines.

### DETAILED DESCRIPTION OF THE INVENTION

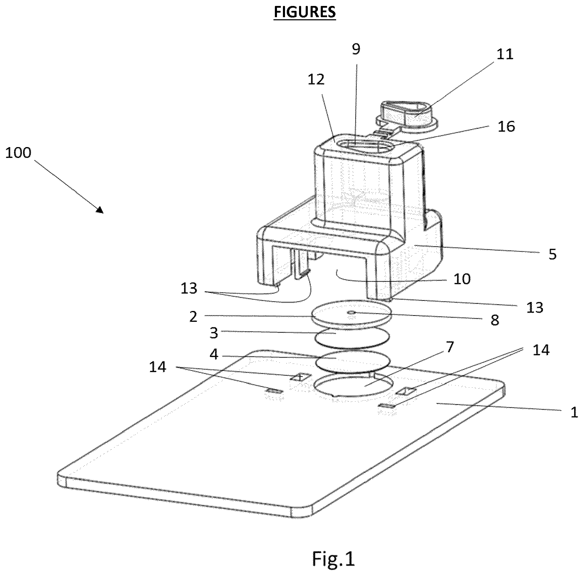

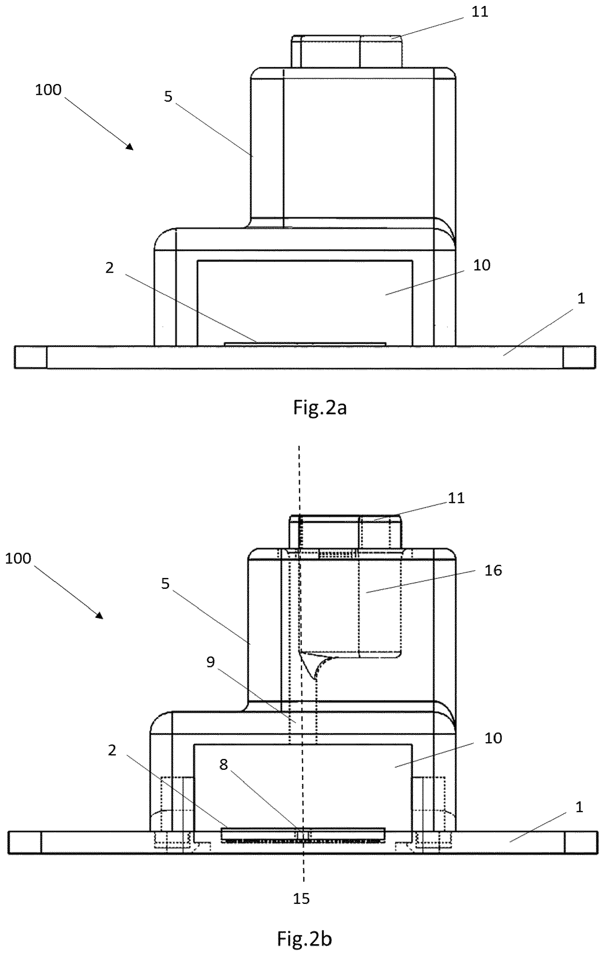

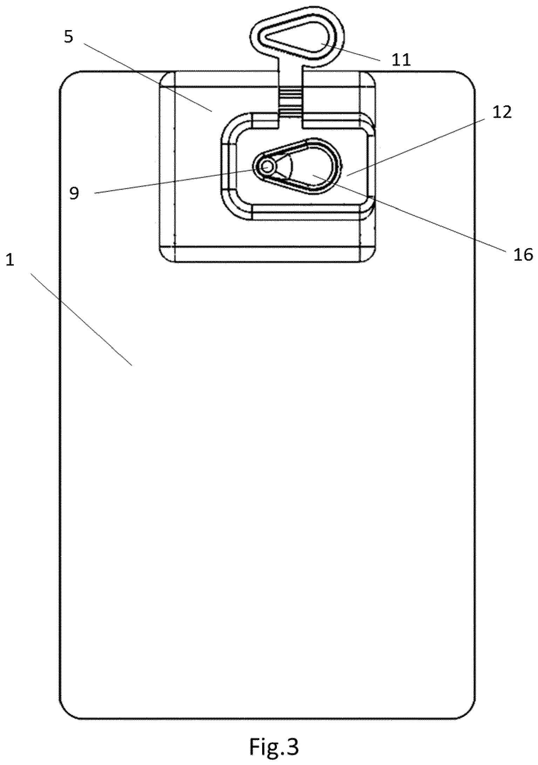

Figure 1 shows an exploded view of an embodiment of the sample holder (100) of the present invention. Figures 2a, 2b and 3 show different views of said embodiment.

In the embodiment depicted in figures 1 to 3 the sample holder (100) comprises:
- A main body (1) comprising a first cavity (7), the first cavity (7) comprising a bottom and an open end.
- A stacked structure made of three layers, where the first layer is a paper test (4), the second layer is a paper filter (3) and the third layer is a sample supporting element (2).
- A cover (5).

Also, the sample holder (100) comprises fixing elements (13, 14), namely four protrusions (13) located in the cover (5) and four recesses (14) corresponding to the protrusions (13) and located in the main body (1). In this embodiment, when the protrusions (13) are housed in the recesses (14), two of the protrusions (13) are configured to align the cover (5) relative to the main body (1) and the other two protrusions (13) and recesses (14) are configured to fix the cover (5) to the main body (1).

As it can be appreciated in figures 1 to 3, in this embodiment the sample holder (100) comprises a tunnel (10) which advantageously allows air flow between the cover (5) and the receiving surface of the sample supporting element (2), thus speeding the drying of the sample. In figures 2a and 2b, said tunnel (10) is clearly shown.

Hereinafter, each element of this embodiment of the sample holder is explained in more detail.

### Main body (1)

In this embodiment the main body (1) has a substantially flat configuration and a substantially rectangular shape with rounded corners. The main body (1) can be made of any material. In a preferred embodiment, the main body (1) is made of an optically transparent material, preferably plastic. The main body (1) being made of optically transparent material advantageously allows seeing through the main body (1) the paper test (4) housed in the first cavity (7).

As it can be appreciated in figure 1, the sample holder (100) comprises four recesses (14) adapted to receive corresponding fixing elements in the form of protrusions (13) arranged in the cover (5). In this embodiment the recesses (14) and protrusions (13) are configured such that when each protrusion (13) is housed in a recess (14) there is no gap between them and the cover (5) is fixed to the main body (1).

Additionally, in one embodiment not shown in the figures, the main body (1) comprises a sticker with an identifier for storing relevant information of the sample. The sticker can be for example a barcode or a QR-code. In other embodiment, the identifier is laser engraved on a surface of the main body (1).

### Stacked structure

In this embodiment the stacked structure comprises three elements: the sample supporting element (2), the paper filter (3) and the test paper (4). The test paper (4) is arranged on the bottom of the first cavity (7), the sample supporting element (2) is arranged at the open end of the first cavity (7) and the paper filter (3) is placed between the test paper (4) and the sample supporting element (2). The sample supporting element (2) comprises a receiving surface made of metal and a first through hole (8) arranged at the receiving surface. In this embodiment the metal is a biocompatible metal. The sample supporting element (2) is arranged in the first cavity (7) with the receiving surface facing outwards and thus being accessible through the open end of the first cavity (7). As visible in figures 2a and 2b, in this embodiment the receiving surface slightly protrudes from the surface of the main body (1) and is parallel to it.

In this embodiment the paper filter (3) is made of a biocompatible absorbent material.

In a preferred embodiment, the sample holder (100) comprises the following features:
- Shape of the sample supporting element (2): round.
- Diameter of the sample supporting element (2): from 10mm to 20mm, preferably, 15 mm.
- Thickness of the sample supporting element (2): from 1 mm to 5mm, preferably, 1 mm.
- Material of the sample supporting element (2): gold-plated brass.
- Thickness of the gold plating: 2µm.
- Finish of the receiving surface: jewellery polish.
- Diameter of the first through hole (8): from 1mm to 5mm, preferably, 1.5mm.
- Position of the first through hole (8): centre of the sample supporting element (2).
- Shape of the paper filter (3): round.
- Diameter of the paper filter (3): from 14mm to 30, preferably, 14 mm.
- Material of the paper filter (3): paper filter commonly used in laboratory.
- Thickness of the paper filter (3): from 10 µm to 60 µm, preferably, 15 µm.
- Shape of the test filter (4): round.
- Diameter of the test filter (4): from 5mm to 15mm, preferably, 14 mm.
- Thickness of the test filter (4): from 10 µm to 30 µm, preferably, 15 µm.

### Cover (5)

In the embodiment shown the cover (5) comprises a second through hole (9), a tunnel (10) and a cap (11). The second through hole (9) is configured such that when the cover (5) is attached to the main body (1), the second through hole (9) and the first through hole (8) are aligned. Tunnel (10) is configured to allow flow of air along the receiving surface of the sample supporting element (2).

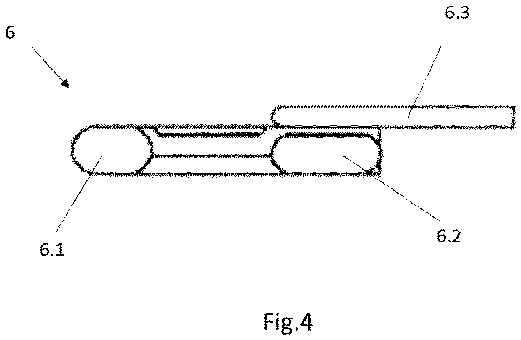

Said cover (5) comprises a surface (12) with a second cavity (16). As shown in figure 3, in this embodiment the opening on the second cavity (16) has a shape of drop of water or pear i.e. said form comprises two rounded ends wherein the angle which defines the aperture of the first rounded end is bigger than the angle which defines the aperture of the second rounded end. The shape of the second cavity (16) is adapted to receive a pipette (6) as shown in figure 4, namely a 20 microliters dual bulb disposable transfer pipette (6) comprising a first (6.1) and a second (6.2) bulb and a transfer tube (6.3). The first end of the opening on the second cavity (16) is configured to house the second bulb (6.2) of the pipette (6) and the second end is configured to house the transfer tube (6.3). As it can be appreciated in figure 2b, wherein the dashed lines represents the internal structure of the cover (5), in this embodiment the second through hole (9) is arranged at the second end of the second cavity (16).

In a particular embodiment, the shape of the second cavity (16) corresponds to the shape of a pipette (6), which advantageously simplifies the insertion and the use of the pipette (6) in the sample holder (100).

In an embodiment of a kit according to the invention, the kit comprises a sample holder as shown in figures 1 to 3 and a 20 microliters disposable transfer pipette (6) as disclosed in figure 4. In a preferred embodiment the cover (5) and the pipette's (6) dimensional features are:
- Diameter of the second through hole (9): 2.2 mm ± 0.1 mm, matching the outer diameter of the transfer tube (6.3) of the pipette (6).
- Height from the delivery end of the transfer tube (6.3) of the pipette (6) to the receiving surface of the sample supporting element (2), when the transfer tube (6.3) is inserted in the cover (5) through the second through hole (9): from 5 mm to 10 mm, preferably 5 mm.
- Height of the tunnel (10): in the range from 7 to 15 mm, preferably 8 mm, wherein 5mm corresponds to the height from transfer tube (6.3) of the pipette (6) to sample supporting element (2) and the remaining 3 mm are configured to save the liquid flowing out of the pipette (6) to adhere to the bottom of the cover (5).

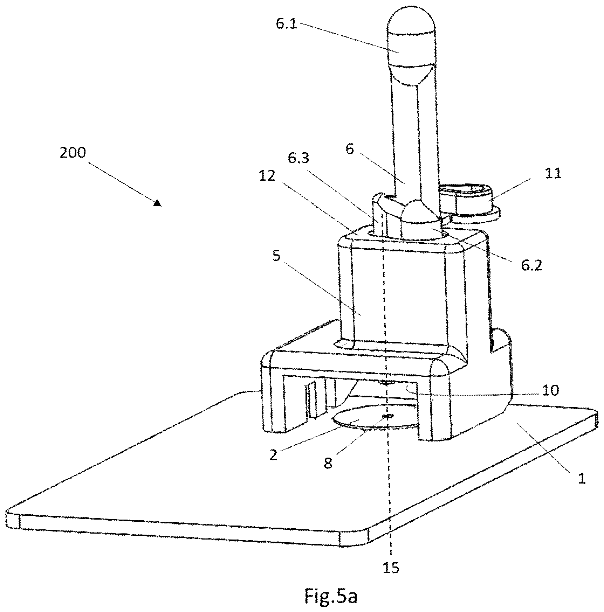

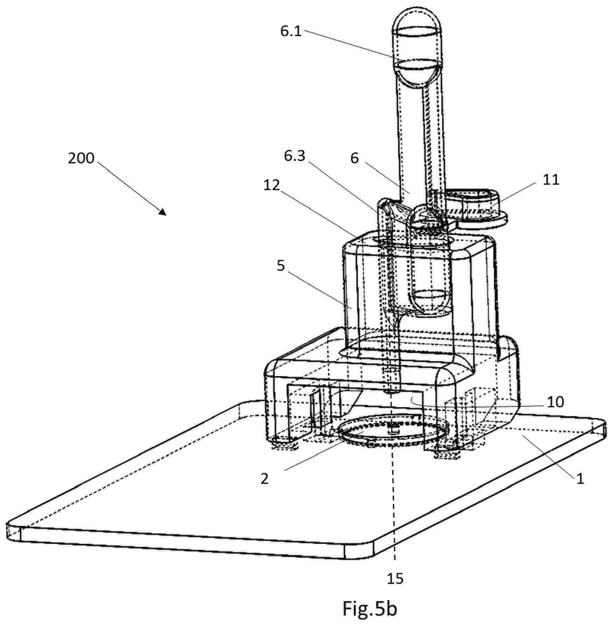

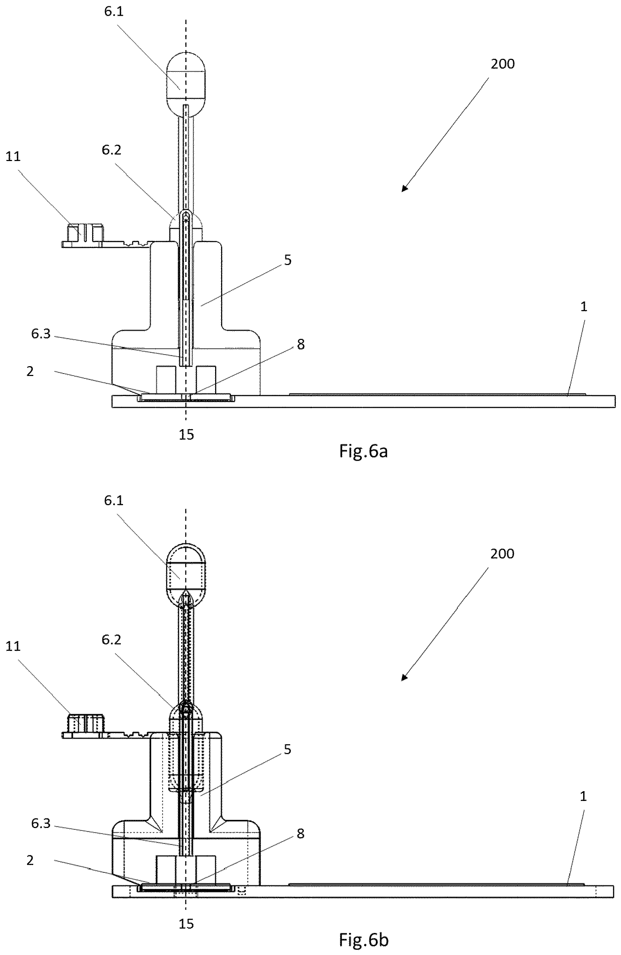

Figure 5a shows a kit (200) according to an embodiment of the present invention. Figure 5b is the same view shown in figure 5a and further includes the internal elements represented in dashed lines in order to show the coupling between the elements of the kit (200) of figure 5a. In particular, said kit (200) comprises the sample holder (100) described in relation to figures 1 to 3 and the pipette (6) shown in figure 4. It can be appreciated that in a use situation of the kit, with the cover (5) fixed to the main body (1) and the pipette (6) housed in the cover (5), the transfer tube (6.3), the second through hole (9) and the first through hole (8) are aligned in the same axis (15), which corresponds to the gravity direction. This alignment is also shown in figures 6a and 6b.

Thus, as the pipette (6) is pressed, the sample drop increases its size and when the drop reaches a sufficiently large diameter the drop is separated from the pipette end due to the action of gravity. When the sample drop is delivered from the pipette (6), the sample drop falls onto the receiving surface at a position including and surrounding the first through hole (8). The liquid sample goes through the first through hole (8) by the action of gravity and capillarity and passes from the receiving surface to the opposite surface of the sample supporting element (2), suctioned by the paper filter (3), which absorbs the sample reaching it. The excess of liquid sample is further absorbed by the test paper (4), which changes colour after getting in contact with this excess of sample, thus providing an indication of the completion of the sample extension. As a result, a thin layer of liquid sample is formed on the receiving surface and the remainder liquid is absorbed by the paper filter (3) and the test paper (4). The sample holder (100) of the invention advantageously allows that the thickness of the sample layer on the receiving surface is very small, which enhances the speed of drying of the liquid sample located on the receiving surface.

### Method for depositing and extending a sample

In a preferred embodiment, the deposition and extension of a sample using the kit (200) of the figures 5a to 6b is accomplished according to the following steps:
1. The sample is loaded into the pipette (6).
2. The transfer pipette is inserted in the second through hole (9) of the cover (5), wherein it is aligned with the first through hole (8).
3. The first bulb (6.1) of the pipette (6) is pressed to deliver the drop of sample. The height from which the drop is delivered avoids reabsorbing the drop after it having touched the receiving surface of the sample supporting element (2). Advantageously, the constant height provided by the cover (5) favours inter-sample deposition repeatability.
4. Once the sample has been deposited onto the receiving surface of the sample supporting element (2), the pipette (6) is extracted from the cover (5) and the cap (11) is plugged.
5. At the same time, the drop on the sample supporting element (2) slowly percolates through the first through hole (8) by capillarity.
6. Once the liquid arrives at the other side of the sample supporting element (2), it touches the filter paper (3) which absorbs the liquid sample until the first through hole (8) of the sample supporting element (2) is emptied. This process lasts from 5 to 10 seconds allowing the sample to be absorbed in a non-violent way, which may change the concentration of specific constituents of the sample (being this sample a biological one).
7. When the paper filter (4) is in contact with the paper test (3), the sample moistens the paper filter (4) which changes colour according to the pH of the liquid sample.
8. The result on the exposed side of main body (1) is a coffee-ring-like distribution of sample with an outer diameter in the order of the few millimetres and a thickness in the order of the tenths of micrometres.
9. After a time of at most 5 minutes exposed to room environmental conditions, i.e. 25 °C, the remaining sample is dried and fixed to the receiving surface of the supporting element (2), ready to be analysed.

## Claims

1. A sample holder (100) comprising:
a main body (1) comprising a first cavity (7), the first cavity (7) comprising a bottom and an open end,
a sample supporting element (2) comprising a receiving surface made of metal, the sample supporting element (2) having a first through hole (8) arranged at the receiving surface, and
a paper filter (3),
wherein the sample supporting element (2) and the paper filter (3) are stacked inside the first cavity (7) of the main body (1), the sample supporting element (2) being arranged on the paper filter (3) and such that the receiving surface of the sample supporting element (2) is accessible through the open end of the first cavity (7).

2. The sample holder (100) according to claim 1, further comprising a cover (5) attachable to the main body (1), the cover (5) comprising a second through hole (9) and being configured such that when the cover (5) is attached to the main body (1), the second through hole (9) and the first through hole (8) are aligned.

3. The sample holder (100) according to claim 2, wherein the cover (5) is configured such that when the cover (5) is attached to the main body (1) the second through hole (9) is kept spaced from the first through hole (8) and wherein the cover (5) further comprises a tunnel (10) configured to allow flow of air along the receiving surface of the sample supporting element (2).

4. The sample holder (100) according to any of claims 2 to 3, wherein the cover (5) further comprises a cap (11) configured to be removably plugged to the second through hole (9).

5. The sample holder (100) according to any of claims 2 to 4, wherein the cover (5) and/or the main body (1) further comprise at least one fixing element (13, 14) configured to attach the cover (5) to the main body (1).

6. The sample holder (100) according to any of the previous claims, further comprising a test paper (4) configured to change its colour when the test paper (4) is in contact with a sample, wherein the test paper (4) is stacked inside the first cavity (7), the paper filter (3) being placed between the test paper (4) and the sample supporting element (2), such that the test paper (4) faces the bottom of the first cavity (7).

7. The sample holder (100) according to claim 6, wherein the test paper (4) is substantially round-shaped and its diameter is in the range from 5 mm to 15 mm.

8. The sample holder (100) according to any of the previous claims, wherein at least a part of the main body (1) corresponding to the first cavity (7) is made of transparent material, such that the paper filter (3) and/or the test paper (4) housed in the interior of the first cavity (7) are visible through the main body (1).

9. The sample holder (100) according to any of the previous claims, wherein the receiving surface is made of a biocompatible metal, wherein preferably the biocompatible metal is gold, aluminium, brass, copper, titanium or stainless steel.

10. The sample holder (100) according to any of the previous claims, wherein the sample supporting element (2) is substantially round-shaped and has a diameter in the range from 10 mm to 20 mm.

11. The sample holder (100) according to any of the previous claims, wherein the paper filter (3) is substantially round-shaped, and has a diameter in the range from 14 mm to 30 mm.

12. The sample holder (100) according to any of the previous claims, wherein one or several of the following conditions are fulfilled:
the thickness of the paper filter (3) is in the range from 10 micrometers to 60 micrometers,
the thickness of the sample supporting element (2) is in the range from 1 mm to 5 mm,
the diameter of the first through hole (8) is in the range from 1 mm to 5 mm.

13. Kit comprising a sample holder (100) according to any of the previous claims and a pipette (6).

14. The kit according to claim 13, wherein the sample holder (100) is according to any of claims 1 to 12 and claim 2, and wherein the cover (5) of the sample holder (100) and the pipette (6) are configured such that when the pipette (6) is inserted in the second through hole (9) of the cover (5) the distance between the delivery end of the pipette and the sample receiving surface is in the range from 5mm to 10mm.

15. Method for depositing and extending a sample comprising the following steps:
a) providing a kit according to claim 13 or 14, when the sample holder (100) is according to any of claims 1 to 12 and claim 2,
b) loading the sample in the pipette (6),
c) inserting the pipette (6) in the second through hole (9) of the cover (5),
d) pressing the pipette (6) to deliver a drop of sample,
e) once the drop of sample has been deposited onto the receiving surface of the sample supporting element (2), extracting the pipette (6) from the cover (5),
f) waiting until the first through hole (8) is emptied and the sample remaining on the receiving surface is dried.
